# EUROPEAN PATENT APPLICATION

(11) **EP 2 098 863 A1**
(43) Date of publication of application: **09.09.2009**
(21) Application number: 08152268.2
(22) Date of filing: 04.03.2008
(51) Int. Cl.: G01N 33/487

(54) **A bio-monitoring system and methods of use thereof**

(71) Applicant: Visgeneer, Inc., Hsinchu City 300 (TW)
(72) Inventor: Dai, Ken-Shwo, 300, Hsinchu City (TW); Wang, Yi-Kai, 300, Hsinchu City (TW)
(74) Representative: Epping - Hermann - Fischer

(57) **Abstract**

A bio-monitoring system used for the determination of analyte in a liquid sample, comprising (1) a meter and a test strip integrated using RFID, wherein the RFID comprises a RFID reader and a RFID tag, wherein the RFID tag is embedded in the test strip or attached to the test strip vial/container, and the RFID tag stores the calibration parameters and strip type information of the test strip; and (2) a radio transmission process system.

## Description

### FIELD OF THE INVENTION

The invention relates to a bio-monitoring system. Particularly, the invention relates to a bio-monitoring system integrated using radio frequency identification (RFID) and methods of use.

### BACKGROUND OF THE INVENTION

Over the past years, many bio-monitoring systems have been developed for quantitative determination of analyte in a liquid sample. The analyte can be glucose, uric acid, cholesterol, triglyceride, glutamyl oxaloacetic transaminase (GOT), glutamyl pyrubic transaminase (GPT) and etc. The technologies applied to the bio-monitoring system for the measurement of analyte concentration can be colorimetry or electrochemistry.

Of the bio-monitoring systems currently in the market, the blood glucose monitoring system has been used daily and proved to be an important tool for diabetic patients to improve their glycemic control. Many characteristics of the bio-monitoring system have been modified to better benefit the users. These modifications include the size of the meter, the size of the strip, the volume of liquid sample, the measuring time, the number of data sets stored in the memory, data transferable, voice synthesizer, data analysis software and etc.

The monitoring system includes a meter and a test strip. In combination with a test strip, the meter is used to calculate and display the quantitative result of analyte in a liquid sample when the liquid sample is applied on the test strip. Since test strips may vary from batch to batch, calibration parameters and strip type information are required for use by the meter in order to obtain accurate reading. These calibration parameters and strip type information can be stored in the meter or in a pluggable code card (U.S. Pat. No. 5,366,609, U.S. Pat. No. 6,780,645 B2 and U.S. Pat. No. 6,814,844 B2). If calibration parameters and strip type information are stored in a pluggable code card, users have to insert the code card into the meter and match the code shown on the LCD with the code labeled on the vial of the test strips before measuring. If calibration parameters and strip type information are pre-stored in the meter, users have to select the code number from the meter to match the code labeled on the vial of the test strips before measurement.

Since the bio-monitoring system has been used as a home-care daily tool to monitor analyte level in body liquid sample (e.g., blood sample), accuracy of the measurement is one of the most critical concerns. There are many factors that can affect the accuracy of the measurement. One of the factors is the user's error during the operation sequence. For example, many users forget to change the code when a new vial of strips (different batch of strips) is used. To address this problem, a reliable, convenient, and intelligent bio-monitoring system is required.

### SUMMARY OF THE INVENTION

According to the present invention, a reliable, convenient, and intelligent bio-monitoring system for quantitative determination of analyte in a liquid sample is provided. The two components (meter and test strip) of this bio-monitoring system is integrated using RFID. The RFID contains two parts which are RFID reader and RFID tag. The meter is installed with a RFID reader whereas the test strip is embedded with a RFID tag or the test strip vial/container is attached with a RFID tag. The RFID tag stores the calibration parameters and strip type information of the test strips. When a measurement is performed, the concentration of analyte in a liquid sample calculated by the meter is based on the calibration parameters and strip type information stored in the RFID tag.

According to one aspect of the present invention, a transmission process system of the bio-monitoring system is provided comprising a meter installed with a RFID reader and a test strip embedded with a RFID tag or the test strip vial/container attached with a RFID tag. The radio transmission process system is applied to send commands from the micro-controller of the meter to the RFID reader; to send radio wave from the RFID reader to the RFID tag embedded in the test strip or attached to test strip vial/container; to send the calibration parameters and strip type information from the RFID tag to the RFID reader, and to send the calibration parameters and strip type information from the RFID reader to the micro-controller.

According to another aspect of the present invention, a meter is provided comprising micro-controller, RFID reader, programmable strip sensing circuit, switch circuit, temperature sensor, in-system programming circuit, and LCD.

According to another aspect of the present invention, a test strip is provided comprising a few base plates, a reaction area and etc. Of the base plates is embedded with a RFID tag.

According to another aspect of the present invention, methods of operation are provided comprising either a 2-step process or a 3-step process. The 2-step operation process comprises (1) insert a test strip into a meter, and (2) apply liquid sample on the test strip. The 3-step operation process comprises (1) insert a test strip into a meter, (2) put the test strip vial /container close to the meter, and (3) apply liquid sample on the test strip.

According to another aspect of the present invention, methods of turning on a meter are provided comprising mechanical or electrical approaches.

### BRIEF DESCRIPTION OF THE DRAWINGS

FIG.1 is a transmission process diagram of the bio-monitoring system according to the present invention.
FIG.2 is a block diagram of the meter according to the present invention.
FIG.3 is a flow diagram illustrating a procedure for determining the concentration of analyte in a liquid sample using test strips embedded with RFID tag.
FIG.4 is a flow diagram illustrating a procedure for determining the concentration of analyte in a liquid sample using test strip vial/container attached with a RFID tag.

### DETAILED DESCRIPTION OF THE INVENTION

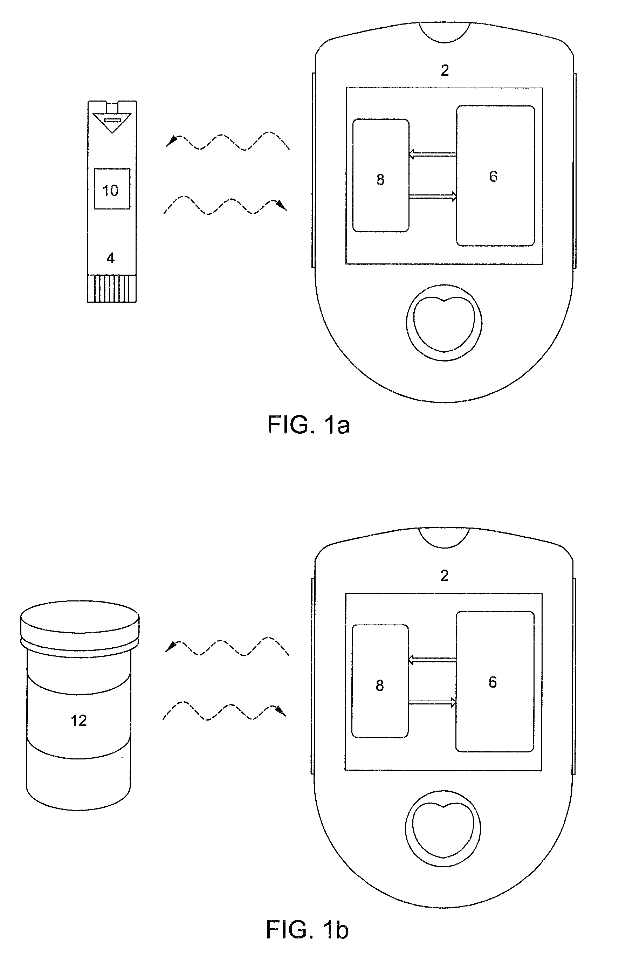

The preferred embodiment of the present invention is shown in FIGS. 1-4. FIG. 1 illustrates a transmission process diagram of the bio-monitoring system according to the present invention. The bio-monitoring system includes a meter 2 and a test strip 4. After the meter 2 is turned on by the test strip 4, a micro-controller 6 of the meter 2 sends command to a RFID reader 8 which is installed in the meter 2. The RFID reader transmits radio wave to a RFID tag 10 embedded in the test strip 4 as shown in Fig. 1a, or attached to the test strip vial/container 12 as shown in Fig. 1b. After receiving the radio wave, the calibration parameters and strip type information of the test strip stored in the RFID tag is then sent back to the RFID reader 8. The micro-controller 6 then reads the calibration parameters and strip type information of the test strip 4 from the RFID reader 8. Using the calibration parameters and strip type information, the micro-controller 6 calculates the input signal generated from the test strip 4 using either colorimetric or electrochemical approaches. Since test strips 4 may vary from batch to batch, the calibration parameters and strip type information of the strip are required in order to obtain accurate analyte reading. From the bio-monitoring systems currently available on the market, the calibration parameters are stored in a pluggable code card or in the meter 2. As stated previously, the present invention provides a bio-monitoring system that eliminates the code-matching procedure which is time consuming and error prone.

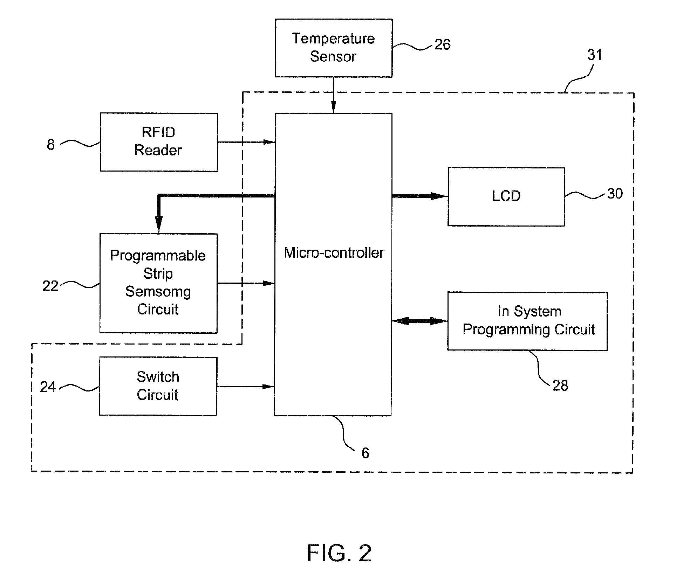

Fig. 2 illustrates a block diagram of the meter according to the present invention. The meter comprises a micro-controller 6, a RFID reader 8, a programmable strip sensing circuit 22, a switch circuit 24, a temperature sensor 26, an in-system programming circuit 28, and a LCD 30. This meter is designed as an all-in-one device which is capable of performing single or multi-analyte test. The micro-controller 6 provides overall control of the meter. After turning on the meter by the strip, the micro-controller 6 reads the calibration parameters and strip type information from RFID tag, and then sends command to the programmable strip sensing circuit 22 to select the reference voltage and current gain which is suitable for the strip type (e.g., glucose strip, uric acid strip, cholesterol strip and etc.). The micro-controller 6 can also read the input from the temperature sensor 26. The switch circuit 24 connects to a switch used for memory and for adjusting time, etc. The LCD 30 is used to show information such as result, date, testing time, message symbol and etc. The in-system programming circuit 30 is used to renew or upgrade software. The dashed line area represents a share area 31. This share area 31 is a common design in many portable electronic device such as mobile phone, personal digital assistant (PDA), digital photo frame, and digital camera, indicating that these portable electronic devices can be used as the meter for the bio-monitoring system of the present invention.

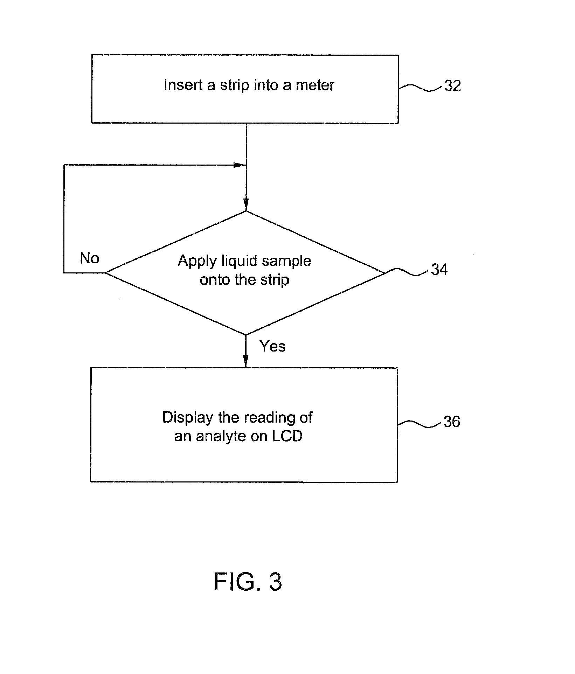

Fig. 3 shows a flow diagram illustrating a 2-step procedure for determining the concentration of analyte in a liquid sample using strips embedded with RFID tag. The process comprises two main steps as follows:
The first step is to insert a test strip into a meter 32. This step is to turn on the meter electrically or mechanically; to set the test strip in the place ready for applying liquid sample 34; and to let the meter read the calibration parameters and strip type information of the test strip.
The second step is to apply a liquid sample on the strip. If this operation is successful, the reading of an analyte is displayed on the LCD 36. If this operation is not successful, the operation process of the aforementioned first step is repeated by using a new strip.

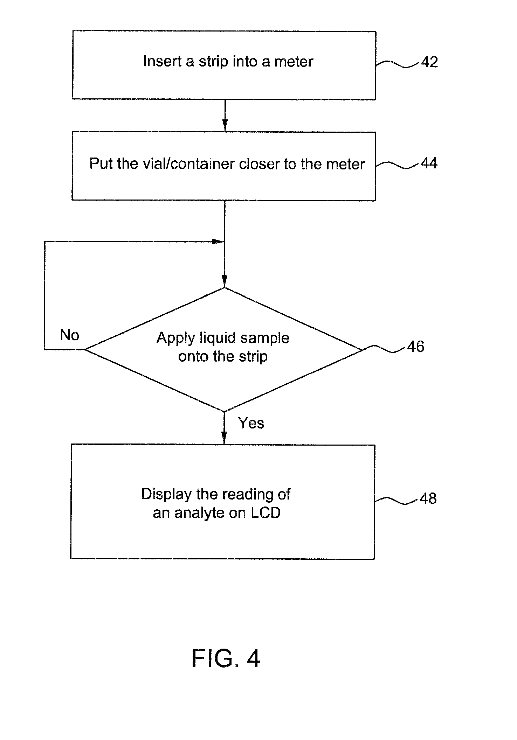

Fig.4 shows a flow diagram illustrating a 3-step procedure for determining the concentration of analyte in a liquid sample using test strip vial/container attached with RFID tag. The process comprises three main steps: the first step is to insert a test strip into a meter 42. This step is to turn on the meter electrically or mechanically, and to set the test strip in the place ready for applying liquid sample.

The second step is to put the test strip vial/container close to the meter 44. This step is to let the meter read the calibration parameters and strip type information of the test strip.

The third step is to apply a liquid sample on the strip 46. If this operation is successful, the reading of an analyte is displayed on the LCD 48. If this operation is not successful, the operation process of the aforementioned second step is repeated by using a new strip.

It will be appreciated by those skilled in the art that changes could be made to the examples described above without departing from the broad inventive concept thereof. It is understood, therefore, that this invention is not limited to the particular examples disclosed, but it is intended to cover modifications within the spirit and scope of the present invention as defined by the appended claims.

## Claims

1. A bio-monitoring system used for the determination of analyte in a liquid sample, comprising:
a meter and a test strip integrated using RFID, wherein the RFID comprises a RFID reader and a RFID tag; wherein
the RFID tag is embedded in the test strip or attached to the test strip vial/container, wherein the RFID tag stores the calibration parameters and strip type information of the test strip; and
a radio transmission process system.

2. The bio-monitoring system of claim 1, wherein the meter comprises a micro-controller, a RFID reader, a programmable strip sensing circuit, a switch circuit, a temperature sensor, an in-system programming circuit, and LCD.

3. The bio-monitoring system of claim 1, wherein the meter is in the form of a mobile phone, a personal digital assistant (PDA), a digital photo frame, or a digital camera.

4. The bio-monitoring system of claim 1, wherein the radio transmission process system is applied to send:
commands from the micro-controller of the meter to the RFID reader;
radio wave from the RFID reader to the RFID tag embedded in the test strip or attached to test strip vial/container;
the calibration parameters and strip type information of the test strip from the RFID tag to the RFID reader; and
the calibration parameters and strip type information of the test strip from the RFID reader to the micro-controller.

5. The bio-monitoring system of claim 1, wherein the analyte is selected from the group of: glucose, uric acid, cholesterol, triglyceride, glutamyl oxaloacetic transaminase (GOT), glutamyl pyrubic transaminase (GPT) and the like.

6. The bio-monitoring system of claim 1, wherein the test strip includes a plurality of base plates and a reaction area.

7. The bio-monitoring system of claim 6, wherein one of the test strip with a plurality of base plates is embedded with the RFID tag.

8. The bio-monitoring system of claim 1, wherein the meter is turned on by a mechanical way.

9. The bio-monitoring system of claim 1, wherein the meter is turned on by an electrical way.

10. The bio-monitoring system of claim 2, wherein the programmable strip sensing circuit is used for the selection of reference voltage and current gain.

11. The bio-monitoring system of claim 10, wherein the selection of reference voltage and current gain is designed for use of different strip types.

12. The bio-monitoring system of claim 2, wherein the in-system programming circuit is used to renew or upgrade software.

13. A 2-step operation method of a bio-monitoring system used to determine analyte in a liquid sample, comprising:
inserting a test strip into a meter:
to turn on the meter electrically or mechanically;
to set the test strip in the place ready for applying liquid sample; and
to let the meter read the calibration parameters/information of the test strip;
applying liquid sample on the strip, wherein
if this operation is successful, the reading of an analyte is displayed on LCD;
if this operation is not successful, said step of inserting a test strip into a meter is repeated by using a new strip.

14. A 3-step operation method of a bio-monitoring system used to determine analyte in a liquid sample, comprising:
inserting a test strip into a meter:
to turn on the meter electrically or mechanically; and
to set the test strip in the place ready for applying liquid sample;
putting the test strip vial/container close to the meter so as to let the meter read the calibration parameters and strip type information of the test strip;
applying liquid sample on the strip, wherein
if this operation is successful, the reading of an analyte is displayed on LCD;
if this operation is not successful, said step of inserting a test strip into a meter is repeated by using a new strip.
